# EUROPEAN PATENT SPECIFICATION

(11) **EP 3 487 445 B1**
(45) Date of publication and mention of the grant of the patent: **13.05.2020**
(21) Application number: 17740411.8
(22) Date of filing: 24.07.2017
(51) Int. Cl.: A61C 17/02, C02F 1/461, A61N 1/44

(54) **ADMINISTRATION OF ORAL CARE ANTIMICROBIALS**
VERABREICHUNG VON ORALEN ANTIMIKROBIELLEN MITTELN
ADMINISTRATION DE SOINS BUCCAUX ANTIMICROBIENS

(30) Priority: 22.07.2016 EP 16180898
(43) Date of publication of application: 29.05.2019
(73) Proprietor: Koninklijke Philips N.V., 5656 AG Eindhoven (NL)
(72) Inventor: DARCH, Owen, Matthew, 5656 AE Eindhoven (NL)
(74) Representative: Marsman, Albert Willem
(86) International application number: PCT/EP2017/068611
(87) International publication number: WO 2018/015570

(56) References cited:
- US-A1- 2006 241 546
- US-A1- 2013 327 353

## Description

### FIELD OF THE INVENTION

The invention is in the field of oral care, and pertains to the generation and administration, into the oral cavity of a subject, of oral care antimicrobials. Particularly, the invention pertains to an integrated system for oral irrigation and administration of electrochemically activated solutions (ECAS).

### BACKGROUND OF THE INVENTION

Dental plaque is a biofilm or mass of bacteria that grows on surfaces within the mouth. It is commonly found between the teeth and along the cervical margins. Dental plaque is also known as microbial plaque, oral biofilm, dental biofilm, dental plaque biofilm or bacterial plaque biofilm. While plaque is commonly associated with oral diseases such as caries and periodontal diseases (gum diseases), such as periodontitis and gingivitis, its formation is a normal process that cannot be prevented.

Dental plaque can give rise to dental caries (tooth decay) - the localised destruction of the tissues of the tooth by acid produced from the bacterial degradation of fermentable sugar - and periodontal problems such as gingivitis and periodontitis. Its progression and build up is what leads to oral problems, hence it is important to disrupt the mass of bacteria and remove it daily.

It is customary to control and remove plaque by means of tooth brushing and interdental aids such as dental floss or oral irrigators.

Removal of dental biofilm is important as it may become acidic causing demineralisation of the teeth (also known as caries), initiate inflammation in the gum, or harden into calculus (also known as tartar). Calculus cannot be removed through toothbrushing or with interdental aids and can only be removed through professional cleaning. Therefore, removal of the dental biofilm will prevent the development of caries and gum diseases.

A persistent problem is that biofilm removal only addresses biofilm once it has grown on a surface. Methods have therefore been developed to reduce the number of live bacteria in the mouth, and particularly on dental surfaces and in interdental spaces. Reducing the number of live bacteria will increase the time before plaque accumulates.

One method to accomplish this is to administer into the oral cavity so-called electrochemically activated solutions (ECAS), sometimes also referred to as electrolysed water. These solutions are based on a process in which antimicrobials are generated by passing a current through a salt solution. Particularly, in such a process a current passes between, e.g., titanium or carbon electrodes to generate the reactive chlorine species hypochlorite (ClO-) and hypochlorous acid (HClO). These species are antimicrobial, and processes for generating them are well known.

Document US2006/0241546 discloses a device for treating burns by administering an oxidative reduction potential (ORP) water solution which is stable for at least twenty-four hours.

### SUMMARY OF THE INVENTION

The invention is as defined in the appended claims.

ECAS generation is common in situations, such as disinfecting swimming pools, wherein no direct concern exists as to health effects of ECAS. In the event of administration into the mouth, however, a too high concentration of hypochlorite will be risky or painful to the subject. Also, in some jurisdictions limits apply to the concentrations to be administered to humans (e.g. in the US a regulation establishes 500 ppm as the maximum residual concentration of hypochlorite allowed in food). At the same time, it is not useful to generate and store ECAS in advance, since it is prone to degradation and will thus lose its activity. Further, particularly since oral care application will generally be carried out in private home environments, conceivably in many different hygienic circumstances, a challenge resides in providing a system that is safe irrespective of differing qualities of input liquids. Particularly, it would be desired to provide a system that is capable of detecting the presence of contaminants in the liquid after ECAS generation, and preferably, thereby adjusting the output of the system.

In order to better address the foregoing desires, the invention provides an oral irrigator system comprising an inlet for liquid, downstream of said inlet a liquid processing section configured to generate a processed output liquid, said processing section comprising an outlet in fluid communication with a dispensing nozzle, said dispensing nozzle having an exit configured to dispense processed liquid into an outside environment, wherein the liquid processing section comprises an electrolysis chamber configured to electrochemically treat a passing liquid and having an outlet for treated liquid, said electrolysis chamber comprising a pair of electrodes connected to a power source, and wherein the liquid processing section comprises, downstream of the electrolysis chamber, a hypochlorite sensor and an oxidation-reduction potential sensor, with said outlet for treated liquid being in fluid communication with said sensors.

The disclosure also presents a method for dental cleaning, the method comprising providing an aqueous solution of sodium chloride, subjecting said solution to electrolysis so as to produce an aqueous hypochlorite/hypochlorous acid solution, and dispensing said hypochlorite/hypochlorous acid solution into the oral cavity, wherein the electrolysis and the dispensing are conducted using an oral irrigator system as described in the preceding paragraph. The dental cleaning can be interdental cleaning.

In another aspect of the disclosure, there is provided a hypochlorite/hypochlorous acid solution for use in dental cleaning, wherein:
- the hypochlorite/hypochlorous acid solution is provided by electrolysis of an aqueous solution of sodium cloride using an oral irrigator system according to any one of the preceding claims, and
- the hypochlorite/hypochlorous acid solution is dispensed into the oral cavity using an oral irrigator system according to any one of the preceding claims.

The solution can be for use in interdental cleaning. The dispensing of the solution is preferbly done not long after it has been made using the electtrolysis, because generally such solutions deteriorate over time. Thus storing time of the solution after the electrolysis is generally less than a few hours or an hour, and preferably less than 15 minutes. More prefereably, the dispensing is done directly after electrolysis.

The hypochlorite/hypochlorous acid solution preferably has an amount of hypochlorite less than 500 ppm.

### BRIEF DESCRIPTION OF THE DRAWINGS

Fig. 1 is a schematic drawing of an oral irrigator in accordance with an embodiment of the invention.
Fig. 2 presents a scheme for a control arrangement of ECAS generation in accordance with an embodiment of the invention.
Fig. 3 is a schematic drawing of an oral irrigator in accordance with another embodiment of the invention.

### DETAILED DESCRIPTION OF THE EMBODIMENTS

In a general sense, the disclosure is based on the judicious insight to combine the *in situ* generation of ECAS with a set of sensors that allows controlling the output purity when using poorly defined input liquids or liquid mixtures. This is accomplished, according to the disclosure, by measuring both the output of hypochlorite and the oxidation reduction potential of the output liquid.

The term "ECAS" refers to any electrochemically activated solution. More particularly, the ECAS is an electrochemically activated solution of sodium chloride. Any source of chloride anion could be used for this reaction, but sodium chloride is used here as an example. As a result of the electrochemical activation, the solution (hereinbefore and hereinafter also referred to as the "hypochlorite/hypochlorous acid solution") comprises a mixture of hypochlorite anions and hypochlorous acid (i.e., a mixture of oxidised chloride ions, with the ratio depending on the surrounding pH of the medium). As customary in the art, ECAS can also be referred to as "electrolysed water." The concentration of sodium chloride prior to electrolysis generally is in a range of from 0.05% to 35.9% (w/v), such as from 0.1% to 10%, preferably from 0.1% to 0.9% (w/v). Apart from sodium chloride solutions, also other salt solutions can be electrochemically activated. Examples hereof include potassium chloride, calcium chloride.

The oxidation-reduction potential (ORP) is a measure of the level of oxidative chemical (or, as applicable reducing chemicals), which are present within a liquid. Hypochlorite itself makes the ORP of a liquid more positive, because it oxidises molecules which are present in the solution. However, other chemicals present in such liquid, particularly if these are also oxidative, will also affect the ORP value of the liquid. In the event of a liquid being subjected to electrolysis, as is the case with *in situ* ECAS generation, such chemicals can be generated if contaminants are present in the liquid (saline) that is used to generate ECAS.

According to the invention, measuring both the concentration of hypochlorite with a specific sensor and the ORP value (which is not specific to hypochlorite), allows an estimation of the level of contamination of an ECAS solution made. Such contaminants are generally undesirable, particularly in the event of a delicate treatment as in oral healthcare, and would otherwise be generated by a device unknowingly.

The invention allows avoiding or reducing the undesirable contaminants in the in situ generated ECAS, by adjusting the electrolysis reaction depending on the determined output of these contaminants. This can be conducted manually, but preferably the oral irrigator is adapted so as to allow the ECAS output to be adjusted automatically. To this end, in a generally preferred embodiment, the liquid processing section comprises a flow rate adjuster upstream of the electrolysis chamber, wherein the hypochlorite and ORP sensors are configured to send feedback to a processor adapted to control the flow rate adjuster. The processor will be set such that in the event of detection of an undesirable level of contaminants. This level will generally be determined in advance by setting a desired level for the hypochlorite output, and a desired level of ORP consistent with the hypochlorite output. In the event that the ORP sensor determines an ORP value higher than that consistent with the hypochlorite output, the processor will instruct the flow rate adjuster to reduce flow rate to reduce production, or increase flow rate to clean the interior of the system, or provide an indication to the user that the device requires cleaning.

It will be understood that the flow rate can also be adjusted in the event that both the hypochlorite output and the ORP value are above a desired level. This provides a general safety measure to avoid an undesirably high output of ECAS itself.

The hypochlorite and ORP sensors can be positioned in series, in either order. In that event, the ECAS output liquid will first pass one sensor (e.g., the hypochlorite sensor) and then the other sensor (*in casu* the ORP sensor). In order to safeguard either of the measurements from affecting the other, it is preferred to position the hypochlorite and ORP sensors in parallel. In that event the ECAS output liquid will be split into two parallel streams. One such stream passes the hypochlorite sensor, and it then sent to the dispensing nozzle. The other stream passes the ORP sensors, and is then sent to the dispensing nozzle. The streams can be re-joined upstream of the dispensing nozzle or inside of the dispensing nozzle. Alternatively, two separate dispensing nozzles are provided, one for either stream. In that event the streams are not re-joined prior to being dispensed, or not re-joined at all (if the two dispensing nozzles are used sequentially).

The oral irrigator system of the invention can be of any type. Various types of oral irrigators exist, ranging from relatively simple mouthwash dispensers, to devices from which pressurized mixtures of liquid and gas are dispensed in the form of jets of high velocity. The system of the invention can include all of its components in a single, hand-held device. The system can also provide part of the components in the form of a base station, and part of the components in a hand-held device that typically can be loaded onto the base station. E.g., the base station will typically comprise a water reservoir (i.e., a liquid container), which is suitable for multiple uses, and which can be easily refilled. The reservoir can be releasable or not. From the reservoir, water can be supplied to a dispensing unit, such as an oral irrigator or a mouthwash dispenser, also held on the base station. In the event of an oral irrigator system comprising a base station, the electrolysis chamber preferably is contained in the base station, but it can also be part of the dispensing unit. The dispensing unit can be a separate dispensing device (such as a hand-held oral irrigator), but it can also be an integrated into the system.

In a further generally preferred embodiment, the invention provides for the *in situ* generation of ECAS, and the aforementioned determination of hypochlorite and ORP output, in a modern oral irrigator, of the type from which jets of relatively small amounts of liquid (preferably including air) are dispensed at relatively high jet velocities. Thereby the oral irrigator system of the invention comprises a dispensing unit, i.e., a hand-held oral irrigator, that allows controlling the actual dispensing of the ECAS through a mixing chamber having a gas inlet that is in fluid communication with a source of pressurized gas and having an outlet to the dispensing nozzle. Thereby the mixing chamber is configured to transport a mixture of liquid and gas to the dispensing nozzle, and whereby the dispensing nozzle is configured to dispense said mixture of liquid and gas to said outside environment.

Preferably, in this embodiment, the oral irrigator system of the invention, in any of the embodiments described above, comprises a liquid container configured to hold a liquid; said container has an outlet for liquid that is in fluid communication with a liquid inlet of a mixing chamber; said mixing chamber having a gas inlet that is in fluid communication with a source of pressurized gas and having an outlet to a dispensing nozzle, whereby the mixing chamber is configured to transport a mixture of liquid and gas to the dispensing nozzle; said dispensing nozzle having an exit to an outside environment, and whereby the dispensing nozzle is configured to dispense said mixture of liquid and gas to said outside environment; wherein the oral irrigator comprises an electrolysis chamber configured to electrochemically treat a passing liquid, said electrolysis chamber comprising a pair of electrodes connected to a power source, wherein the electrolysis chamber is positioned downstream of the liquid container, and in fluid communication therewith, and upstream of the outlet of the mixing chamber, and in fluid communication therewith, and wherein fluid communication from the mixing chamber to the exit of the dispensing nozzle is controllable independently of the fluid communication from the liquid container to the electrolysis chamber.

The term "fluid communication" refers to any connection between a first unit, or a first part of a device, and a second unit, or a second part of a device, via which fluids, including liquids and gases, can flow. Such flow can be direct or indirect. Direct flow can be, e.g., through a fluid communication channel such as a pipe, a tube, a hose, or a flow line, said communication channels possibly comprising one or more valves or other units that serve to open or close the communication channel, or that adjust the size of an opening or closure in said communication channel. Indirect flow can be, e.g., via one or more treatment units, pressure-reducing or increasing units, or other units in which the fluid is subjected to a treatment (such as a chemical reaction or a physical treatment such as mixing or standing). Generally, such units have an inlet for the fluid to enter and an outlet for the fluid to exit.

The fluid communication can be controllable fluid communication. In that event, the direct or indirect flow can be interrupted or adjusted (such as in terms of the absolute amount of liquid, the flow rate, or both). Such interruption or adjustment can be controlled manually, automated, or made dependent on related events, such as feedback information resulting from operating one part of a device, that is sent to a processor adapted to control fluid communication.

The terms "upstream" and "downstream" are used such as to be related the normal operation of an oral irrigator. Accordingly, the downstream side of the oral irrigator system is the exit (such as the exit of a dispensing nozzle) through which an irrigation fluid can be applied to the oral cavity. The units and communication channels that, in the normal operation of the oral irrigator, precede the actual exiting of the irrigation fluid, are thus positioned upstream of the exit.

In a preferred embodiment, the oral irrigator system of the invention serves to dispense, through a jet or a spray, a mixture of liquid and gas. To this end the oral irrigator system comprises a dispensing device (such as an oral irrigator) that comprises a mixing chamber. The mixing chamber has a gas inlet that is in fluid communication with a source of pressurized gas. The source of pressurized gas can be a container (such as a gas canister) adapted to hold pressurized gas. In that event, such container has a gas outlet that is in fluid communication with a gas inlet of the mixing chamber. Suitable gases are, e.g., carbon dioxide, nitrogen, or pressurized air. Particularly, such gases can be of medical grade.

In an interesting embodiment, the source of pressurized gas refers to a compression unit, such as a pump configured to forward pressurized gas into the mixing chamber. In an embodiment thereof, the pump is adapted to draw gas, particularly air, from an outside environment into a gas inlet of the oral irrigator, prior to pressing same into the mixing chamber. Thereby the pump preferably is a reciprocating pump, such as a plunger pump or a piston pump. E.g., the pump comprises a motor adapted to suck air into said gas inlet, whereby the gas inlet is in fluid communication with a piston. The piston is generally contained in a piston chamber configured to allow backward and forward movement of the piston (typically a cylinder).

Typically, thereby air will be drawn into both the piston chamber and the mixing chamber. However, a provision can also be made to close off the mixing chamber from the air inlet, and allow an inlet into the mixing chamber to be opened upon release of the piston.

The piston is able to be released from a locked position, such that, upon release, it will press the air into the mixing chamber. The release of the piston typically follows after a desired amount of air has been drawn into the piston chamber, and desirably compressed. It will be understood that drawing air into the piston will typically refer to drawing air into a chamber, such as a cylinder, that on one end (e.g., a longitudinal end, such as the bottom of the chamber) is closed off by the piston, and on an opposite end (e.g. the opposite longitudinal end, such as the top of the chamber) is in fluid communication with the mixing chamber, or is in fluid communication with the mixing chamber upon release of the piston. The piston can be released by various mechanisms. E.g., manually or as a result of a desired, e.g. predetermined, amount of air having been let in. Appropriate releasable fixations for a piston are known to the skilled person, such as a lock that can be pushed or drawn away, a spring underneath the piston that exerts a releasing action dependent on the amount of air drawn onto the piston, or other triggers available in the art.

Compression units, e.g. micro-compressors, suitable for being included in oral irrigator systems, particularly in hand-held type oral irrigators, are well available to the skilled person. Without being limitative, reference can be made to background descriptions in WO 02/1372, US 2010/35200, WO2015/173691.

Without wishing to be bound by theory, the inventors believe that, in addition to the controlling of contaminants as described above, the invention strikes a balance between the need to generate ECAS *in situ,* so as to prevent untimely degradation of ECAS, and a sufficient residence time of a salt solution in an electrolysis chamber to generate a desirable amount of ECAS.

To this end, the fluid communication from the mixing chamber to the exit of the dispensing nozzle is controllable independently of the fluid communication from the liquid container to the electrolysis chamber. As a result, the residence time of the salt solution during electrolysis in the electrolysis chamber can be adjusted.

In an interesting embodiment of the oral irrigator system of the invention, when comprising an oral irrigator of the type dispensing a mixture of liquid and gas, the electrolysis chamber is positioned upstream of the mixing chamber, and in controllable fluid communication therewith. This facilitates the aforementioned possibility to adjust the residence time of liquid (such as a salt solution) in the electrolysis chamber. Also, an advantage is that an oral irrigation fluid can be dispensed from the mixing chamber irrespective of whether or not an amount of ECAS is included. In addition to the latter, any desired flexibility of dispensing fluids with or without ECAS can be further increased by the addition of one or more further liquid containers. Such further liquid containers can be adapted to hold water and/or solutions or dispersions comprising oral care agents other than ECAS.

In another interesting embodiment, the mixing chamber comprises the electrolysis chamber. Particularly, the electrolysis chamber and the mixing chamber thereby coincide. In this embodiment, the electrolysis effectively takes place in the mixing chamber. This has an advantage in that the oral irrigator system of the invention can be a hand-held oral irrigator just as compact as a conventional oral irrigator without an electrolysis chamber. Another advantage is that the setting of the residence time of liquid in the electrolysis chamber and the frequency of dispensing oral irrigation fluid require a single setting only, which simplifies the operation of the device by the end-user. In this embodiment it is preferred for the electrodes to be provided each on a wall of the mixing chamber, so as to provide a physically undisturbed mixing environment.

It will be understood that various other embodiments are possible. E.g., in the embodiment in which the electrolysis is conducted in the mixing chamber, the device of the invention can comprise one or more additional liquid containers adapted to supply the mixing chamber with other liquids. Or, e.g., in the embodiment in which the electrolysis chamber is positioned upstream of the mixing chamber, it will be possible to include such control electronics in the device, that the electrolysis, the mixing, and the dispensing are adapted relative to each other such as to present a single, optimal setting to the end-user. Also, in any of the embodiments, it is conceivable to include such controls that the electrolysis can be set "on" or "off." This can be either through manual operation, or via a pre-programmed choice of settings.

Preferably, the release, into the mixing chamber, of pressurized gas, also serves to control the fluid communication from the mixing chamber to the exit of the dispensing nozzle. I.e., the mixing chamber and the dispensing nozzle are designed such that liquid contained in the mixing chamber is not dispensed, unless and until pressurized gas is made to enter the mixing chamber, and to exit the dispensing nozzle. This does not exclude that, in some embodiments, the air inlet itself is in fluid communication with the mixing chamber.

As a result of encountering the pressurized gas, the liquid in the mixing chamber will mostly break up into a plurality of droplets. Some of the liquid will thereby remain in a stream form, and some of the gas will remain in a streaming form, all furthered into the dispensing nozzle, and forced (as a result of the gas pressure) through the exit thereof.

As mentioned above, a challenge in providing ECAS specifically for oral care, is to control the ECAS concentration dispensed into the oral cavity. The oral irrigator of the invention allows the implementation of a system to this end. Thereby, the device of the invention comprises a flow rate adjuster adapted to adjust the flow rate of liquid from the liquid container into the electrolysis chamber. A flow rate adjuster can be, e.g., a valve through which the amount of liquid per unit of time entering the electrolysis chamber can be set, e.g., by adjusting the size of an aperture through which the liquid enters said chamber. Also other flow rate adjusters are conceivable, e.g. the addition of a circular fluid communication loop provided with an adjustable pump, via which the flow rate of the liquid can be set directly.

The flow rate adjuster of the invention is applied in conjunction with at least the hypochlorite and ORP sensors. Preferably, a saline sensor upstream of the electrolysis chamber is provided that measures the input into the electrolysis chamber, in addition to the output from the electrolysis chamber being measured by the hypochlorite and ORP sensors.

The hypochlorite and ORP sensors, as well as saline sensors, can be provided by the skilled person as available in the art. The sensors are preferably adapted to send feedback to a processor that, in turn, is adapted to control the flow rate adjuster. Non-limiting examples of hypochlorite sensors include amperometric sensors, such as provided by Dosatronic, e.g., DOSASens type DCL10, Chlorine Sensor Type CP 2.1, Chlorine Sensor Type CS 2.3, or the FCLTX-100 series from Omega. Also beyond these specific examples, hypochlorite sensors can be provided without further research. Examples of ORP sensors include, but are not limited to, Sensorex S1500C-ORP Light-Duty Polycarbonate ORP (REDOX) Sensor, Vernier ORP Sensor, PASS™ TestSafe ORP Meter, Osmotics HM Digital ORP-200: Waterproof ORP Meter, Hach® IntelliCAL™ MTC101 Rugged Gel Filled ORP Electrode.

As indicated above, the oral irrigator system of the invention preferably comprises a salinity sensor upstream of the flow rate adjuster. This sensor too, is adapted to send feedback to a processor adapted to control the flow rate adjuster. Also a salinity sensor, which measures salt concentration, can be provided as available in the art. Suitable examples can be found in existing technology using conductance measurements, similar to pool chlorination measurements.

The electrolysis chamber itself can be designed in a known way. Generally two electrodes are present, connected to a power source so as to provide an anode and a cathode. The electrodes can, each independently, be made of any suitable material, such as iron, carbon, platinum or any other electrical conductor material. In the invention it is preferred to use carbon or titanium, or a combination of each. Electrodes can suitably be coated, e.g. to improve lifespan. Examples of coatings include mixed metal oxide (MMO) coatings containing various components e.g. iridium, ruthenium, platinum, zirconium, niobium, tantalum. Preferably, the electrodes are plate electrodes. These can be flat plates, extending into the electrolysis chamber or put against the walls thereof. Particularly in the latter case, the electrodes can be curved, e.g., following the shape of the wall of an electrolysis chamber provided in a hand-held oral irrigator.

The power source can be obtained from an AC connection, such as a standard domestic power supply, typically employing an adapter so as to convert domestic AC power to DC power. The power source preferably is a battery, or a set of batteries, preferably placed in a corresponding compartment of the oral irrigator system (such as in a base station, or in a hand-held oral irrigator, or both). The batteries can be replaceable and/or rechargeable, as is customary in the art, particularly for hand-held oral irrigators. In one embodiment, the system of the invention, particularly in the form of a dispensing device, comprises a battery compartment having a conductive connection to the electrode system. In another embodiment, the device comprises both an adapter for a domestic AC outlet, and a battery compartment, both of which having a conductive connection to the electrode system. Particularly in the event that the electrolysis chamber is part of a base station, it is preferred for such base station to be connectable to a domestic AC outlet. More particularly, such separate units to be held on the base station and requiring power, such as a hand-held oral irrigator, can be recharged on the base station.

An oral irrigator system, such as an interdental cleaner, typically comprises a source of liquid; a system for moving a selected amount of liquid from the source thereof into a liquid pathway; a driving unit such as a pump or a source of pressurized gas, or a combination thereof; and a control arrangement for releasing a selected amount of gas into contact with the liquid, resulting in liquid being propelled out of a nozzle portion of the cleaner. Suitable devices are described, *inter alia,* in WO 2010/055433, WO 2010/055434, WO 2008/012707, WO 2014/068431. Typically preferred jet velocities are of the order of 5 m/s to 50 m/s, such as 10 m/s to 40 m/s, such as 20 m/s to 30m/s. The pumping unit or units can be separate compressors, the pumping function can be provided by the pressurized gas, or both. Preferably, the oral irrigator system comprises a microburst pump, typically contained in a dispensing unit such as a hand-held oral irrigator.

The oral irrigator of the invention can be in the form of a system in which part of the components can be hand-held, and part of the components can be comprises in or on a basis or docking station. Preferably, the oral irrigator of the invention is fully hand-held. In such a device, the components of the oral irrigator system as described above are all contained in or on a housing that is adapted (taking into account size, shape, and weight) to be capable of being normally lifted and held by a human, preferably in a single hand.

The disclosure also pertains to a method for interdental cleaning. The method comprises providing an aqueous solution of sodium chloride, subjecting said solution to electrolysis so as to produce an aqueous hypochlorite solution, and dispensing said hypochlorite solution into the oral cavity, wherein the electrolysis and the dispensing are conducted by means of an oral irrigator system as described hereinbefore, in all if its embodiments.

The oral irrigator system of the invention dispenses an oral irrigation fluid, preferably comprising a mixture of liquid and gas, particularly pressurized air. Hitherto, in the art, such a fluid has not been provided in conjunction with ECAS. In this respect the disclosure also pertains to an oral irrigation fluid comprising water, sodium hypochlorite, and air.

The invention will further be illustrated with reference to the non-limiting figures discussed hereinafter.

In the drawings the uninterrupted lines indicate flow of liquid, the broken lines indicate flow of information, including control operations. In the drawings, the following components are shown:
(2) A pump configured to forward pressurized air ;
(3) Communication channels (tubes) for fluid;
(4) A (typically optional) valve;
(5) A pressure chamber (mixing chamber);
(6) A nozzle for dispensing oral irrigation fluid into the oral cavity, particularly of a human;
(7) An electrolysis chamber (ECAS cell);
(8) A feeding pump for liquid to be subjected to electrolysis;
(9) Control electronics;
(10) Data connections for transferring information;
(11) A salinity sensor;
(12) A flow rate adjuster;
(13) A processor;
(14) A hypochlorite sensor;
(15) An oxidation reduction potential (ORP) sensor;
(16) A base station;
(17) A reservoir for liquid;
(18) A hand-held oral irrigator;

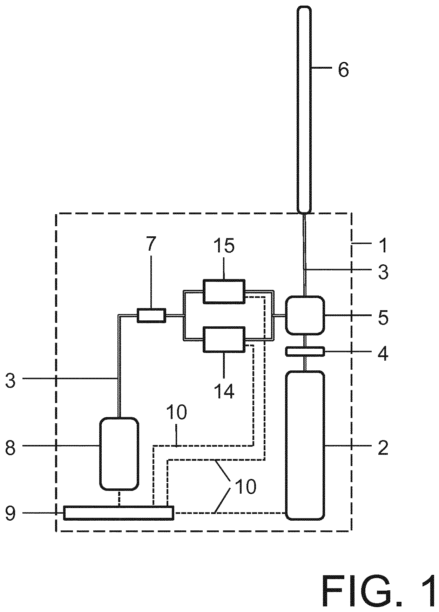

Fig. 1 is a schematic drawing of an oral irrigator system (1) in accordance with a preferred embodiment of the invention. Herein a system is shown in which all of the components of the invention are part of a single oral irrigator device. With reference to the above components, the device allows water supplied to it (not shown) to be fed by a pump (8), via a flow line (3), to the electrolysis chamber (7). The output from the electrolysis chamber, i.e., ECAS, is subjected to both hypochlorite measurement and oxidation reduction potential measurement, by the corresponding sensors, respectively (14) and (15). The measurement information obtained from these sensors is sent to a set of control electronics (9) by which the feeding of water to the electrolysis chamber (7) and/or the functioning of the electrolysis chamber can be controlled. Not shown, but generally applicable to the invention in all of its embodiments, is a control mechanism in the event that the simultaneous output of the sensors reveals an undesirable amount of contaminants. In that event, irrespective of whether or not the ECAS generation is reduced, a control mechanism can also entail the changing of the supply of water into the system, e.g., by switching from one source (such as tap water) to another source (such as bottled water).

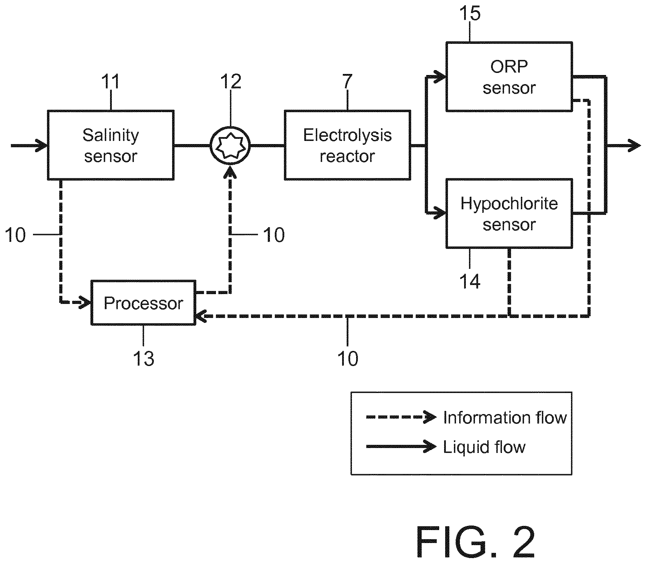

Fig. 2 presents a scheme for a control arrangement of ECAS generation in accordance with an embodiment of the invention. Herein the uninterrupted lines indicate flow of liquid, the broken lines indicate flow of information. Liquid to be electrolysed (a) flows to an electrolysis chamber (7) via a salinity sensor (11) and via a flow rate adjuster (12), positioned downstream of the salinity sensor, and upstream of the electrolysis chamber. Electrolysed liquid (d) passes, in parallel, through a hypochlorite sensor (14), respectively an ORP sensor (15), before being dispensed. Information (b) obtained from the salinity sensor is processed in a processor (13); Information (e) and (f) obtained from the hypochlorite and ORP sensors is also processed in the processor, thus providing a feedback loop in relation to the amount of ECAS generated. Information (c) from the processor is sent to the flow rate adjuster, so as to adjust the flow rate of liquid to be electrolysed depending on the processed data related to salinity input and hypochlorite output.

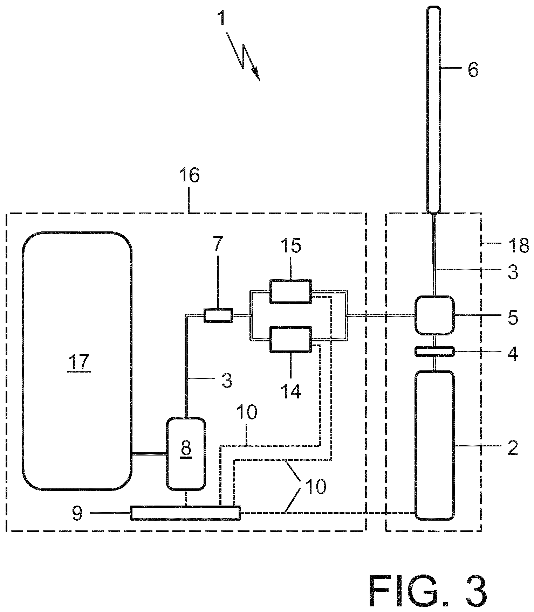

Fig. 3 is a schematic drawing of an oral irrigator system (1) in accordance with another preferred embodiment of the invention. Herein the system comprises a separate dispensing device (18), such as a hand-held oral irrigator, and a base station (16) typically configured so as to hold the dispensing device, and possibly (not shown) any other devices or units that can be used in providing oral care, such as an electronic toothbrush. In the embodiment shown, the base station also comprises a reservoir for liquid (i.e., a liquid container).

While the invention has been illustrated and described in detail in the drawings and foregoing description, such illustration and description are to be considered illustrative or exemplary and not restrictive; the invention is not limited to the disclosed embodiments.

For example, it is possible to operate the invention in an embodiment wherein further sensors are present (downstream of the electrolysis chamber), so as to measure other quality characteristics. Also, it is conceivable to arrange for only one the sensors to send feedback to a processing unit, and the other sensor to be read out manually. In embodiments having a base station and a separate dispensing unit, it is also possible for the electrolysis to neither be contained in the base station, nor in the dispensing unit, but to be present as a separate unit to be held on the base station.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain features of the invention are recited in mutually different dependent claims does not indicate that a combination of these features cannot be used to advantage. Any reference signs in the claims should not be construed as limiting the scope.

In sum, we hereby disclose is a set-up for an oral irrigator, as a system of components and preferably as a single, hand-held device. Herein a provision is made to dispense oral irrigation fluid comprising antimicrobial ions/chemical species produced *in situ* (in the system or device) by electrolysis of an appropriate salt solution, i.e., an electrochemically activated solution (ECAS). The production of ECAS is enabled by providing an electrolysis chamber. The ECAS output liquid is controlled by means of two different sensors, viz. a hypochlorite sensor and an ORP (oxidation reduction potential) sensor. The sensors allow the determination of undesirable contaminants after ECAS generation, by determining a possible difference in the increase (or decrease) of the values for hypochlorite and ORP.

## Claims

1. An oral irrigator system (1) comprising an inlet for liquid, downstream of said inlet a liquid processing section configured to generate a processed output liquid, said processing section comprising an outlet in fluid communication with a dispensing nozzle (6), said dispensing nozzle (6) having an exit configured to dispense processed liquid into an outside environment, wherein the liquid processing section comprises an electrolysis chamber (7) configured to electrochemically treat a passing liquid and having an outlet for treated liquid, said electrolysis chamber (7) comprising a pair of electrodes connected to a power source, and wherein the liquid processing section comprises, downstream of the electrolysis chamber (7), a hypochlorite sensor (14) and an oxidation-reduction potential sensor (15), with said outlet for treated liquid being in fluid communication with said sensors (14, 15).

2. An oral irrigator system according to claim 1, wherein the liquid processing section comprises a flow rate adjuster upstream of the electrolysis chamber, and wherein said sensors are configured to send feedback to a processor adapted to control the flow rate adjuster.

3. An oral irrigator system according to claim 1 or 2, wherein the hypochlorite sensor and the oxidation-reduction potential sensor are positioned in parallel.

4. An oral irrigator system according to claim 2 or 3, comprising a salinity sensor upstream of the flow rate adjuster, said sensor adapted to send feedback to a processor adapted to control the flow rate adjuster.

5. An oral irrigator system according to any one of the preceding claims, comprising a mixing chamber; said mixing chamber having a gas inlet that is in fluid communication with a source of pressurized gas and having an outlet to the dispensing nozzle, whereby the mixing chamber is configured to transport a mixture of liquid and gas to the dispensing nozzle; and whereby the dispensing nozzle is configured to dispense said mixture of liquid and gas to said outside environment, and wherein the outlet for treated liquid of the electrolysis chamber is in fluid communication with an inlet of the mixing chamber.

6. An oral irrigator system according to claim 5, comprising a base station, a liquid container, and a hand-held oral irrigator, said liquid container and said oral irrigator being releasably fixed on the base station, wherein the base station comprises the electrolysis chamber and the hand-held oral irrigator comprises the mixing chamber, wherein a liquid outlet of the liquid container is in fluid communication with a liquid inlet of the oral irrigator, and wherein the outlet for treated liquid of the electrolysis chamber is in fluid communication with a liquid inlet of the oral irrigator.

7. An oral irrigator system according to claim 6, wherein the hypochlorite sensor and the oxidation reduction potential sensor are positioned downstream of the outlet for treated liquid of the electrolysis chamber and upstream of the liquid inlet of the oral irrigator.

8. An oral irrigator system according to claim 5, wherein the system is in the form of a hand-held oral irrigator comprising a liquid container configured to hold a liquid; said container having an outlet for liquid that is in fluid communication with a liquid inlet of the mixing chamber, said hand-held oral irrigator comprising the electrolysis chamber, said electrolysis chamber being downstream of the liquid container.

9. An oral irrigator system according to claim 8, wherein the electrolysis chamber is positioned upstream of the mixing chamber, and in controllable fluid communication therewith.

10. An oral irrigator system according to any one of the claims 5 to 9, configured so as to allow the release of pressurized gas into the mixing chamber to serve controlling the fluid communication from the mixing chamber to the exit of the dispensing nozzle.

11. An oral irrigator system according to any one of the claims 5 to 10, wherein the mixing chamber comprises the electrolysis chamber.

12. An oral irrigator according to claim 11, wherein the electrodes are provided on a wall of the mixing chamber.

13. An oral irrigator system according to any one of the claims 5 to 12, wherein the source of pressurized gas is a compression unit, such as a pump, configured to forward pressurized gas into the mixing chamber.

14. An oral irrigator system according to claim 13, comprising an air inlet and wherein the pump is adapted to generate pressurized air, preferably comprising a piston.

## Patentansprüche

1. Mundspülsystem (1), umfassend einen Einlass für Flüssigkeit, stromabwärts des Einlasses einen Flüssigkeitsverarbeitungsabschnitt, der konfiguriert ist, um eine verarbeitete Ausgangsflüssigkeit zu erzeugen, wobei der Verarbeitungsabschnitt einen Auslass umfasst, der mit einer Abgabedüse (6) in Fluidverbindung steht, wobei die genannte Abgabedüse (6) einen Ausgang aufweist, der konfiguriert ist, um verarbeitete Flüssigkeit in eine Außenumgebung abzugeben, wobei der Flüssigkeitsverarbeitungsabschnitt eine Elektrolysekammer (7) umfasst, die konfiguriert ist, um eine vorbeiziehende Flüssigkeit elektrochemisch zu behandeln, und einen Auslass für behandelte Flüssigkeit aufweist, wobei die Elektrolysekammer (7) ein Elektrodenpaar umfasst, das mit einer Stromquelle verbunden ist, und wobei der Flüssigkeitsverarbeitungsabschnitt stromabwärts der Elektrolysekammer (7) einen Hypochloritsensor (14) und einen Oxidations-Reduktions-Potential-Sensor (15) umfasst, wobei der Auslass für die behandelte Flüssigkeit mit den genannten Sensoren (14, 15) in Fluidverbindung steht.

2. Mundspülsystem nach Anspruch 1, wobei der Flüssigkeitsverarbeitungsabschnitt einen Strömungsrateneinsteller stromaufwärts der Elektrolysekammer umfasst, und wobei die Sensoren konfiguriert sind, um eine Rückmeldung an einen Prozessor zu senden, der zur Steuerung des Strömungsrateneinstellers geeignet ist.

3. Mundspülsystem nach Anspruch 1 oder 2, wobei der Hypochloritsensor und der Oxidations-Reduktions-Potential-Sensor parallel positioniert sind.

4. Mundspülsystem nach Anspruch 2 oder 3, umfassend einen Salzgehaltsensor stromaufwärts des Strömungsrateneinstellers, wobei der Sensor dazu ausgelegt ist, um eine Rückmeldung an einen Prozessor zu senden, der zur Steuerung des Strömungsrateneinstellers ausgelegt ist.

5. Mundspülsystem nach einem der vorhergehenden Ansprüche, umfassend eine Mischkammer; wobei die genannte Mischkammer einen Gaseinlass aufweist, der mit einer Druckgasquelle in Fluidverbindung steht, und einen Auslass zur Abgabedüse aufweist, wobei die Mischkammer so konfiguriert ist, dass sie ein Gemisch aus Flüssigkeit und Gas zur Abgabedüse transportiert; und wobei die Abgabedüse so konfiguriert ist, dass sie das Gemisch aus Flüssigkeit und Gas an die Außenumgebung abgibt, und wobei der Auslass für die behandelte Flüssigkeit der Elektrolysekammer in Fluidverbindung mit einem Einlass der Mischkammer steht.

6. Mundspülsystem nach Anspruch 5, umfassend eine Basisstation, einen Flüssigkeitsbehälter und ein tragbares Mundspülgerät, wobei der Flüssigkeitsbehälter und das Mundspülgerät lösbar an der Basisstation befestigt sind, wobei die Basisstation die Elektrolysekammer umfasst, und das tragbare Mundspülgerät die Mischkammer umfasst, wobei ein Flüssigkeitsauslass des Flüssigkeitsbehälters mit einem Flüssigkeitseinlass des Mundspülgeräts in Fluidverbindung steht, und wobei der Auslass für die behandelte Flüssigkeit der Elektrolysekammer mit einem Flüssigkeitseinlass des Mundspülgeräts in Fluidverbindung steht.

7. Mundspülsystem nach Anspruch 6, wobei der Hypochloritsensor und der Oxidations-Reduktions-Potential-Sensor stromabwärts des Auslasses für die behandelte Flüssigkeit der Elektrolysekammer und stromaufwärts des Flüssigkeitseinlasses des Mundspülgeräts angeordnet sind.

8. Mundspülsystem nach Anspruch 5, wobei das System die Form eines tragbaren Mundspülgeräts aufweist, umfassend einen Flüssigkeitsbehälter, der zum Halten einer Flüssigkeit konfiguriert ist; wobei der Behälter einen Auslass für Flüssigkeit aufweist, der mit einem Flüssigkeitseinlass der Mischkammer in Fluidverbindung steht, wobei das tragbare Mundspülgerät die Elektrolysekammer umfasst, wobei die genannte Elektrolysekammer stromabwärts des Flüssigkeitsbehälters ist.

9. Mundspülsystem nach Anspruch 8, wobei die Elektrolysekammer stromaufwärts der Mischkammer positioniert ist und mit dieser in steuerbarer Fluidverbindung steht.

10. Mundspülsystem nach einem der Ansprüche 5 bis 9, das so konfiguriert ist, dass Druckgas in die Mischkammer freigesetzt werden kann, um die Fluidverbindung von der Mischkammer zum Ausgang der Abgabedüse zu steuern.

11. Mundspülsystem nach einem der Ansprüche 5 bis 10, wobei die Mischkammer die Elektrolysekammer umfasst.

12. Mundspülgerät nach Anspruch 11, wobei die Elektroden an einer Wand der Mischkammer vorgesehen sind.

13. Mundspülsystem nach einem der Ansprüche 5 bis 12, wobei die Druckgasquelle eine Kompressionseinheit wie eine Pumpe ist, die konfiguriert ist, um Druckgas in die Mischkammer weiterzuleiten.

14. Mundspülsystem nach Anspruch 13, umfassend einen Lufteinlass, und wobei die Pumpe zur Erzeugung von Druckluft angepasst ist, und vorzugsweise einen Kolben umfasst.

## Revendications

1. Système d'irrigateur buccal (1) comprenant une entrée de liquide, en aval de ladite entrée une section de traitement de liquide configurée pour générer un liquide de sortie traité, ladite section de traitement comprenant une sortie en communication fluidique avec une buse de distribution (6), ladite buse de distribution (6) ayant une sortie configurée pour distribuer le liquide traité dans un environnement extérieur, dans lequel la section de traitement de liquide comprend une chambre d'électrolyse (7) configurée pour traiter électrochimiquement un liquide qui passe et ayant une sortie pour le liquide traité, ladite chambre d'électrolyse (7) comprenant une paire d'électrodes connectées à une source d'alimentation, et dans lequel la section de traitement de liquide comprend, en aval de la chambre d'électrolyse (7), un capteur d'hypochlorite (14) et un capteur de potentiel d'oxydoréduction (15), avec ladite sortie pour le liquide traité qui est en communication fluidique avec lesdits capteurs (14, 15).

2. Système d'irrigation buccale selon la revendication 1, dans lequel la section de traitement de liquide comprend un dispositif de réglage du débit en amont de la chambre d'électrolyse, et dans lequel lesdits capteurs sont configurés pour envoyer un feedback à un processeur adapté pour contrôler le dispositif de réglage du débit.

3. Système d'irrigation buccale selon la revendication 1 ou 2, dans lequel le capteur d'hypochlorite et le capteur de potentiel d'oxydoréduction sont positionnés en parallèle.

4. Système d'irrigateur buccal selon la revendication 2 ou 3, comprenant un capteur de salinité en amont du dispositif de réglage du débit, ledit capteur étant adapté pour envoyer un feedback à un processeur adapté pour contrôler le dispositif de réglage du débit.

5. Système d'irrigateur buccal selon l'une quelconque des revendications précédentes, comprenant une chambre de mélange; ladite chambre de mélange ayant une entrée de gaz qui est en communication fluidique avec une source de gaz pressurisé et ayant une sortie vers la buse de distribution, moyennant quoi la chambre de mélange est configurée pour transporter un mélange de liquide et de gaz vers la buse de distribution; et moyennant quoi la buse de distribution est configurée pour distribuer ledit mélange de liquide et de gaz audit environnement extérieur, et où la sortie pour le liquide traité de la chambre d'électrolyse est en communication fluidique avec une entrée de la chambre de mélange.

6. Système d'irrigateur buccal selon la revendication 5, comprenant une station de base, un récipient de liquide et un irrigateur buccal à main, ledit récipient de liquide et ledit irrigateur buccal étant fixés de manière amovible sur la station de base, dans lequel la station de base comprend la chambre d'électrolyse et l'irrigateur buccal à main comprend la chambre de mélange, dans lequel une sortie de liquide du récipient de liquide est en communication fluidique avec une entrée de liquide de l'irrigateur buccal, et dans lequel la sortie pour le liquide traité de la chambre d'électrolyse est en communication fluidique avec un entrée de liquide de l'irrigateur buccal.

7. Système d'irrigateur buccal selon la revendication 6, dans lequel le capteur d'hypochlorite et le capteur de potentiel d'oxydoréduction sont positionnés en aval de la sortie pour le liquide traité de la chambre d'électrolyse et en amont de l'entrée de liquide de l'irrigateur buccal.

8. Système d'irrigateur buccal selon la revendication 5, dans lequel le système est sous la forme d'un irrigateur buccal à main comprenant un récipient de liquide configuré pour contenir un liquide; ledit récipient ayant une sortie pour le liquide qui est en communication fluidique avec une entrée de liquide de la chambre de mélange, ledit irrigateur buccal à main comprenant la chambre d'électrolyse, ladite chambre d'électrolyse étant en aval du récipient de liquide.

9. Système d'irrigation buccal selon la revendication 8, dans lequel la chambre d'électrolyse est positionnée en amont de la chambre de mélange, et en communication fluidique contrôlable avec celle-ci.

10. Système d'irrigation buccal selon l'une quelconque des revendications 5 à 9, configuré de manière à permettre la libération de gaz pressurisé dans la chambre de mélange pour servir à contrôler la communication fluidique de la chambre de mélange à la sortie de la buse de distribution.

11. Système d'irrigateur buccal selon l'une quelconque des revendications 5 à 10, dans lequel la chambre de mélange comprend la chambre d'électrolyse.

12. Irrigateur buccal selon la revendication 11, dans lequel les électrodes sont prévues sur une paroi de la chambre de mélange.

13. Système d'irrigation buccale selon l'une quelconque des revendications 5 à 12, dans lequel la source de gaz pressurisé est une unité de compression, telle qu'une pompe, configurée pour envoyer du gaz pressurisé dans la chambre de mélange.

14. Système d'irrigation buccale selon la revendication 13, comprenant une entrée d'air et dans lequel la pompe est adaptée pour générer de l'air pressurisé, comprenant de préférence un piston.
